# EUROPEAN PATENT SPECIFICATION

(11) **EP 0 787 121 B1**
(45) Date of publication and mention of the grant of the patent: **26.04.2000**
(21) Application number: 95931447.7
(22) Date of filing: 12.09.1995
(51) Int. Cl.: C07C 69/52, C12P 17/02

(54) **LEGIONELLA SPECIFIC ANTIBIOTIC, A MICROORGANISM PRODUCING THE ANTIBIOTIC AND A PROCESS FOR PRODUCING THE SAME**
LEGIONELLA-SPEZIFISCHES ANTIBIOTIKUM, DASSELBIGE PRODUZIERENDER MIKROORGANISMUS UND VERFAHREN ZU DESSEN HERSTELLUNG
ANTIBIOTIQUE SPECIFIQUE DE LEGIONELLA, MICRO-ORGANISME PERMETTANT D'OBTENIR CET ANTIBIOTIQUE ET SON PROCEDE DE PRODUCTION

(30) Priority: 14.09.1994 KR 9423582; 14.09.1994 KR 9423583; 14.09.1994 KR 9423584
(43) Date of publication of application: 06.08.1997
(73) Proprietor: CHEIL FOODS & CHEMICALS, INC., Seoul 100-095 (KR); KOREA INSTITUTE OF SCIENCE AND TECHNOLOGY, Seoul 136-791 (KR)
(72) Inventor: LEE, Yong, Woo Seonduk Apartment, Seoul 136-104 (KR); LEE, Yeong, Seon Dongshin Apartment, Goyang-city Kyunggi-do 411-020 (KR); YON, Chang, Suek Woosung Apartment, Seoul 121-042 (KR); SUH, Jung, Woo Shindonga Apartment, Inchun-city 402-042 (KR); LEE, Chul, Hoon Woochang Apartment, Seoul 138-130 (KR); LIM, Yoong, Ho, Anyang-city Kyunggi-do 430-053 (KR); YOO, Ick, Dong Hyundai Apartment, Daejeon-city 305-340 (KR)
(74) Representative: Gervasi, Gemma, Dr.
(86) International application number: PCT/KR95/00117
(87) International publication number: WO 96/08460

(56) References cited:
- FR-A- 2 692 579
- CHEMICAL ABSTRACTS, Vol. 112, No. 23, 4 June 1990, (Columbus, Ohio, USA), page 504, Abstract No. 215293q, A.P. PCHELKIN et al., "Chromatographic Study of Diacylglycerol Mixture Obtained by Hydrolysis of Vegetable Oils"; & PRIKL. BIOKHIM. MIKROBIOL., 1988, 24(6), 809-15, (Russ).
- CHEMICAL ABSTRACTS, Vol. 83, No. 9, 1 September 1975, (Columbus, Ohio, USA), page 603, Abstract No. 78516f, M.S. BAINOVA et al., "Synthesis and Properties of Minor Cryptoactive Triglycerides of Cocoa Butter"; & KHIM. PRIR. SOEDIN., 1975, 11(1), 8-11, (Russ).
- CHEMICAL ABSTRACTS, Vol. 123, No. 17, 23 October 1995, (Columbus, Ohio, USA), page 571, Abstract No. 222428t, CH. YON et al., "AL072, A Novel Anti-Legionella Antibiotic Produced by Streptomyces Sp."; & J. ANTIBIOT., 1995, 48(8), 773-9, (Eng).

## Description

The present invention is directed to a novel *Legionella* specific antibiotic AL072, a novel *Streptomyces* sp. AL91 producing the same, and a process for producing the said antibiotic.

### BACKGROUND OF THE INVENTION

*Legionella pneumophila*, which is the causal agent of *Legionella* infections, has been known to cause Legionnaires' disease or Pontiac fever. Since the bacteria was first isolated in Philadelpia, USA, it has been reported that many isolations of a species of *Legionella* were made from various patients and environments in all parts of the world, including the USA, and 10 or more species were identified. *Legionella pneumophila* is one of the pathogens capable of causing pneumonia and it is presumed that 5% of the occurences of pneumonia are due to *Legionella pneumophila*. *Legionella pneumophila* grows in air-conditioning cooling towers, water service pipes, drain pipes, etc., and infections are believed to occur in respiratory organs by inhalation of contaminated aerosols. In July, 1984, criticism was caused by the surprising fact that ill-defined symptoms of pneumonia which occurred in patients as well as medical teams at intensive care units of Koryo Hospital in Seoul appeared to be caused by *Legionella* species.
According to a result of investigation completed in 1985 by the National Health Institute, at least 90% of air-conditioning cooling towers within Seoul were contaminated with *Legionella* species and 93% of the isolated *Legionella* were identified as *Legionella pneumophila.* An additional investigation completed by the National Health Institute in major cities throughout the country, for example Seoul, Pusan, Daejeon, etc., between June and September, 1988 revealed that 83% of the isolated *Legionella* were classified to *Legionella pneumophila* serogroup 1.

Macrolide antibiotics, such as erythromycin, and quinolone antibiotics, such as rifampin, are known to be active against *Legionella* species and have been used for the treatment of the *Legionella* infections. However, these antibiotics have a wide spectrum of activity against a variety of microbes, in addition to the *Legionella* species. In this regard, an abuse of such antibiotics for extended periods may not only generate resistance to various microbes but also cause harmful problems such as the collapse of the balance of microbes occurring in a human body.

Additionally, there have been serious problems in that chemical agents for disinfecting air-conditioning cooling towers, which are contamination sources of *Legionella* species, may result in the contamination of the environment and the corrosion of the air-conditioning device.

Therefore, the development of novel *Legionella* specific antibiotics which are specifically active against only *Legionella* species, is needed and thereby may minimize the undesired problems. We have intensively investigated soil microbes over several years for the purpose of developing such antibiotics. Eventually, we succeeded in isolating a species of *Streptomyces* producing *Legionella* specific antibiotics and purifying a novel antibiotic specifically active against only *Legionella* species, which is designated Antibiotic AL072.

### SUMMARY OF THE INVENTION

Cultivation of the novel microorganism *Streptomyces* sp. AL91 yields a novel antibiotic substance AL072 which is active exclusively against *Legionella* species, whose structure is represented as follows:

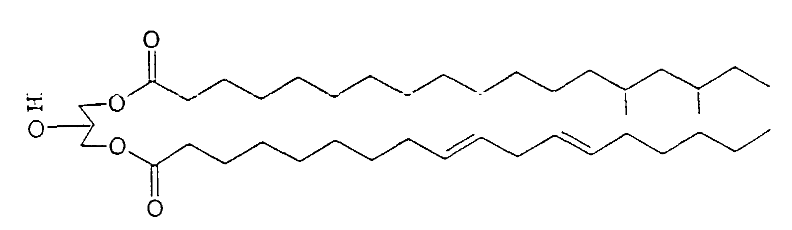

### DESCRIPTION OF THE DRAWINGS

- FIG. 1: shows the ultraviolet absorption spectrum of antibiotic substance AL072.
- FIG. 2: shows the infrared absorption spectrum of antibiotic substance AL072.

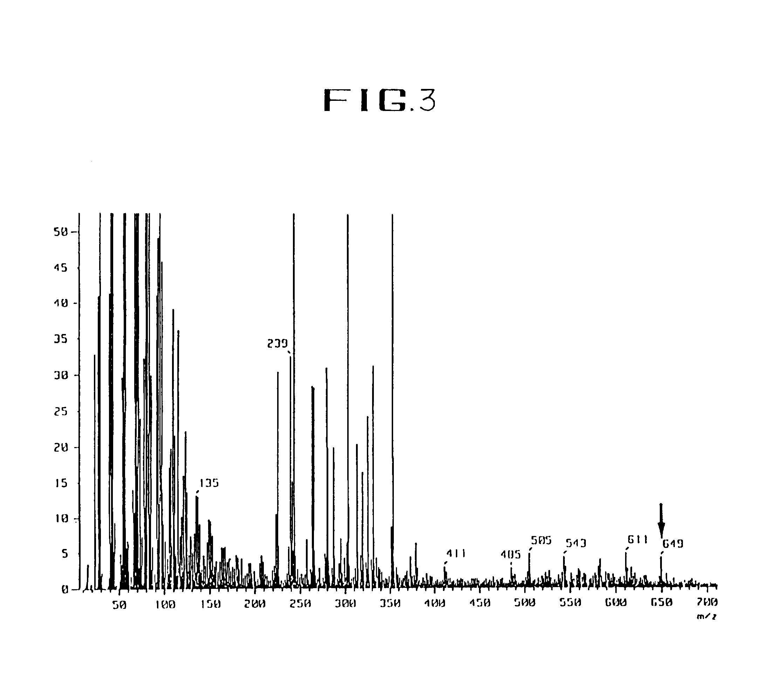

- FIG. 3: shows the mass spectrum of antibiotic substance AL072 measured by FAB/MS.
- FIG. 4: shows the 400 MHz proton unclear magnetic resonance spectrum of antibiotic substance AL072.
- FIG. 5: shows the 100 MHz carbon nuclear magnetic resonance spectrum of antibiotic substance AL072.

### DETAILED DESCRIPTION OF THE INVENTION

### The Microorganism

The microorganism used for the production of *Legionella* specific antibiotic substance AL072 is *Streptomyces* sp. AL91. The microorganism was deposited at the permanent collection of the Korean Culture Center of Microorganisms, Seoul, Korea, on June 2, 1994 under the Budapest Treaty on the international recognition of the deposit of microorganisms for the purposes of patent procedure, and a subculture of the organism can be obtained from the repository under the accession number KCCM 10055. In addition to the specific microorganism described and characterized herein, it should be understood that mutants of the microorganism (e.g., mutants produced through the use of X-ray, ultraviolet radiation or nitrogen mustards) can also be cultivated to produce the antibiotic substane AL072.

Isolation of *Streptomyces* sp. AL91 was accomplished by shaking 1.0g of dried soil sample in 10ml of sterile distilled water, plating 0.1ml of dilutions of the soil suspension on a Humic acid-Vitamin agar supplemented with Cycloheximide (50µg/ml) and Nalidixic acid (200µg/ml), and cultivating the plate at the temperature of 28°C for 14-21 days. The medium was sterilized at 121°C for 15 minutes before the antifungal agent Cycloheximide and the antibacterial agent Nalidixic acid, separately sterilized by filtration, were added. The composition of Humic acid-Vitamin agar is described in Table I.

**Table I**

| | |
|---|---|
| Humic acid (dissolved in 10ml of 0.2 N NaOH) | 1.0g |
| Na₂HPO₄ | 0.5g |
| KCl | 1.71g |
| MgSO₄ · 7H₂O | 0.05g |
| FeSO₄ · 7H₂O | 0.01g |
| Thiamin-HCl | 0.5g |
| Vitamin B₂ | 0.5g |
| Niacin | 0.5g |
| Pyridoxine-HCl | 0.5g |
| Inositol | 0.5g |
| Pantothenic Acid Ca-salt | 0.5g |
| *p*-Aminobenzoic Acid | 0.5g |
| Biotin | 0.25mg |
| Cycloheximide | 50mg |
| Nalidixic acid | 200g/mL |
| Agar | 20g |
| Distilled water(pH 7.0) | 1L |

### Characteristics of Streptomyces sp. AL91 KCCM 10055

- Morphology:: Spores are formed by spiral, branched chains. The surface of spores is smooth.
- Biochemical characteristics:: The ability to liquefy gelatin is negative and the ability to degrade starch is positive.

| Cultural characteristcs: | | | | |
|---|---|---|---|---|
| Media | Growth | Color of aerial mycelium | Reverse color | Soluble pigments |
| Trypton-yeast extract agar | good | brown | brown | brown |
| | | | | |
| Yeast extract-malt extract agar | good | white | brown | - |
| | | | | |
| Oat meal extract agar | good | pale orange or white | - | - |
| | | | | |
| Inorganic salt-starch agar | good | white | - | - |
| | | | | |
| Glycerol-asparagine agar | good | dense yellow | dense yellow | - |
| | | | | |
| Peptone-yeast extract-iron agar | poor | dense brown | brown | dense brown |
| | | | | |
| Tyrosine agar | good | white-brown | dense brown | - |
| | | | | |
| Bennett' medium | good | white | yellow-brown | - |

### Carbon utilization:

- Positive:: D-Glucose, sucrose, D-xylose, D-mannitol, D-fructose, rhamnose, raffinose, cellulose.
- Negative:: L-Arabinose, i-inositol.

| Susceptibility to antibiotics: | | |
|---|---|---|
| antibiotics | Concentrations (µg/mL) | Diameter of ring produced by inhibition of growth (mm) |
| Carbenicillin | 100 | - |
| Chloramphenicol | 30 | 27.7 |
| Neomycin | 30 | 12.0 |
| Nalidixic acid | 30 | - |
| Vancomycin | 30 | 22.0 |
| Clindamycin | 2 | - |
| Ampicillin | 10 | 12.0 |
| Kanamycin | 30 | 17.0 |
| Tetracycline | 30 | 17.0 |
| Cephalothin | 30 | 24.0 |
| Erythromycin | 15 | 40.0 |
| Rifampin | 5 | - |
| Gentamycin | 10 | 10.0 |
| Streptomycin | 10 | 17.0 |

### Production of the Antibiotic substance AL072

*Streptomyces* sp. AL91 KCCM 10055 was grown on a nutrient agar containing the components listed in Table II for 3 days. The cultures were inoculated into 200 mL of a liquid medium containing the components listed in Table III and cultivated at the temperature of 28°C under aerobic conditions for 3 days. Subsequently, the culture solution was inoculated into 6 L of a liquid medium containing the components listed in Table IV and cultivated at the temperature of 28°C under aerobic conditions for 5 days. The antibiotic AL072 was isolated and purified from the final culture solution by the procedures described below.

**Table II**

| | |
|---|---|
| Sucrose | 20.0g |
| Glucose | 10.0g |
| Corn steep liquor | 5mg |
| Yeast extracts | 4.9g |
| Soybean flour | 20.0g |
| CaCO₃ | 4.0g |
| NaCl | 2.0g |
| K₂HPO₄ | 0.05g |
| Agar | 15g |
| Distilled water (pH 7.3) | 1L |

**Table III**

| | |
|---|---|
| Glucose | 1g |
| Soluble starch | 24g |
| Peptone | 3g |
| Malt extract | 5g |
| CaCO₃ | 4g |
| Distilled water (pH 7.0) | 1L |

**Table IV**

| | |
|---|---|
| Sucrose | 20.0g |
| Glucose | 10.0g |
| Corn steep liquor | 5ml |
| Yeast extract | 4.9g |
| Soybean flour | 20.0g |
| CaCO₃ | 4.0g |
| NaCl | 2.0g |
| K₂HPO₄ | 0.05g |
| Distilled water (pH 7.3) | 1L |

After cultivation was completed, 6L of the culture solution was mixed with an equivalent amount of isopropyl alcohol by stirring. After standing over night, the mixture was centrifuged and the resulting supernatant was taken. The supernatant was filtered through the passage of diatomaceous earth and the filtrate was then concentrated under reduced pressure to remove the isopropyl alcohol. The resulting concentrate was three times extracted with ethylacetate, which was then removed under reduced pressure. The residue was dissolved in 50% isopropyl alcohol and the resulting solution was then concentrated under reduced pressure to remove the isopropyl alcohol. The residual aqueous solution was passed on a column filled with octadecyl silica gel and the passed solution was discarded. *Legionella* specific antibiotics adhered to the ODS resin were eluted with 70% ethyl alcohol and the elutes were then concentrated under reduced pressure to dryness. After the dried concentrates were dissolved in 80% isopropyl alcohol, preparative high pressure liquid chromatography (206nm) on sillica gel, eluting with acetonitrile-distilled water, 68 : 32 at the rate of 30mL/min, gave crude *Legionella* specific antibiotic substance AL072. Subsequently, the crude antibiotics were concentrated under reduced pressure todryness, the dried concentrates were dissolved in 50% isopropyl and the resulting solution was again concentrated under reduced pressure. The isopropyl alcohol was removed and the residue was three times extracted with chloroform. The chloroform was removed and the residue was dissolved in 80% isopropyl alcohol. High pressure liquid chromatography, run in the same conditions as used in the first chromatography, gave pure antibiotic AL072.

### Physico-chemical properties of Antibiotic substance AL072

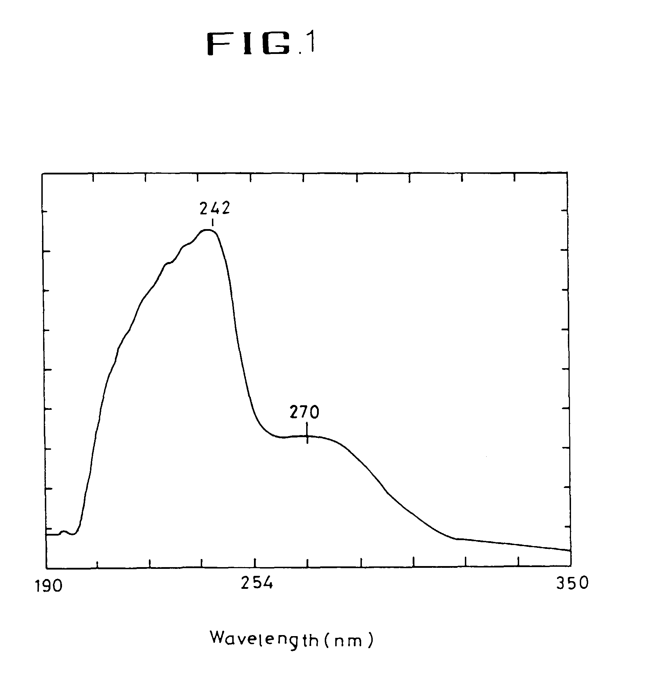

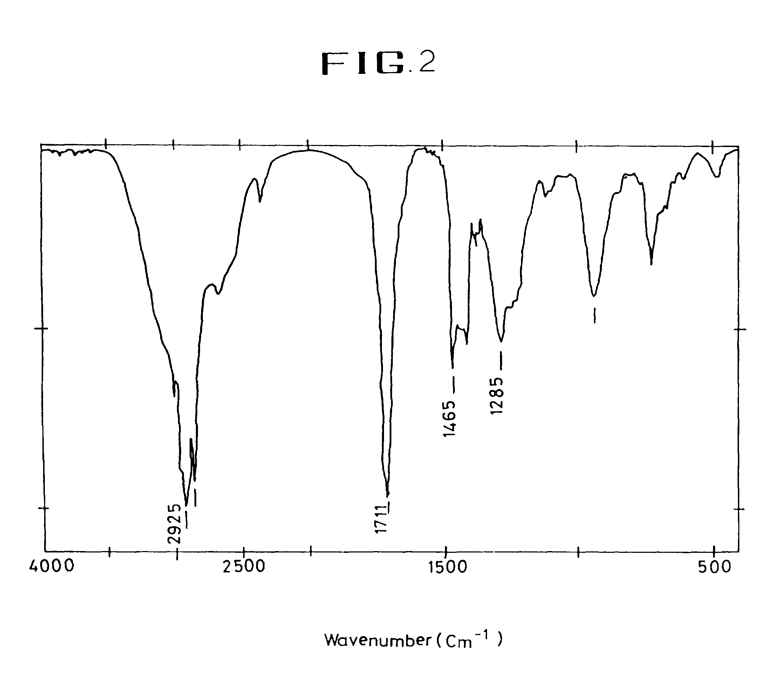

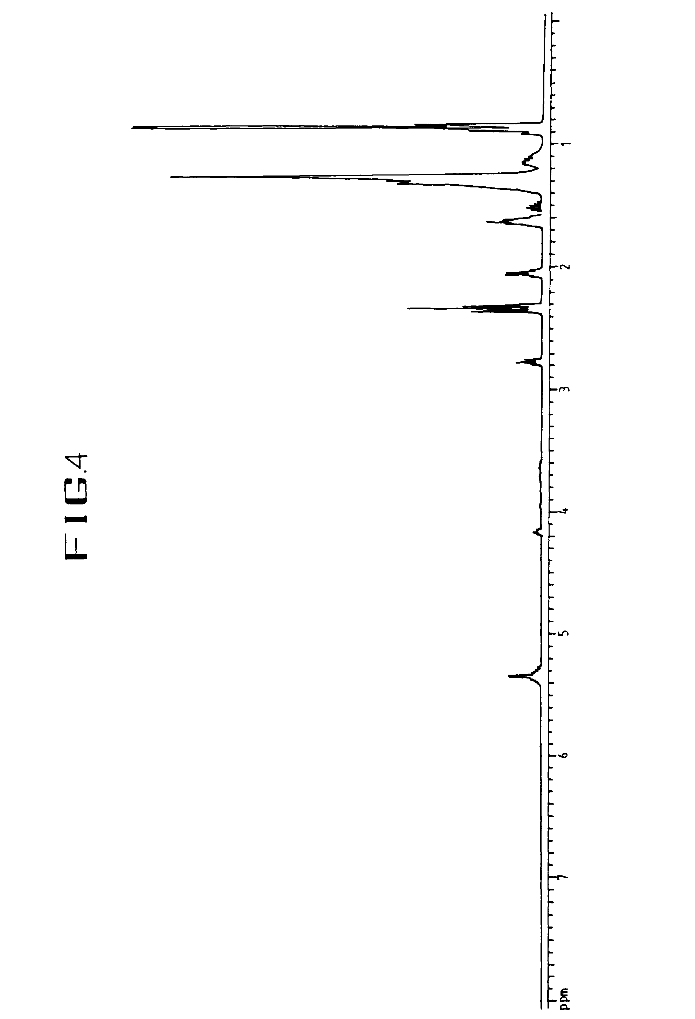

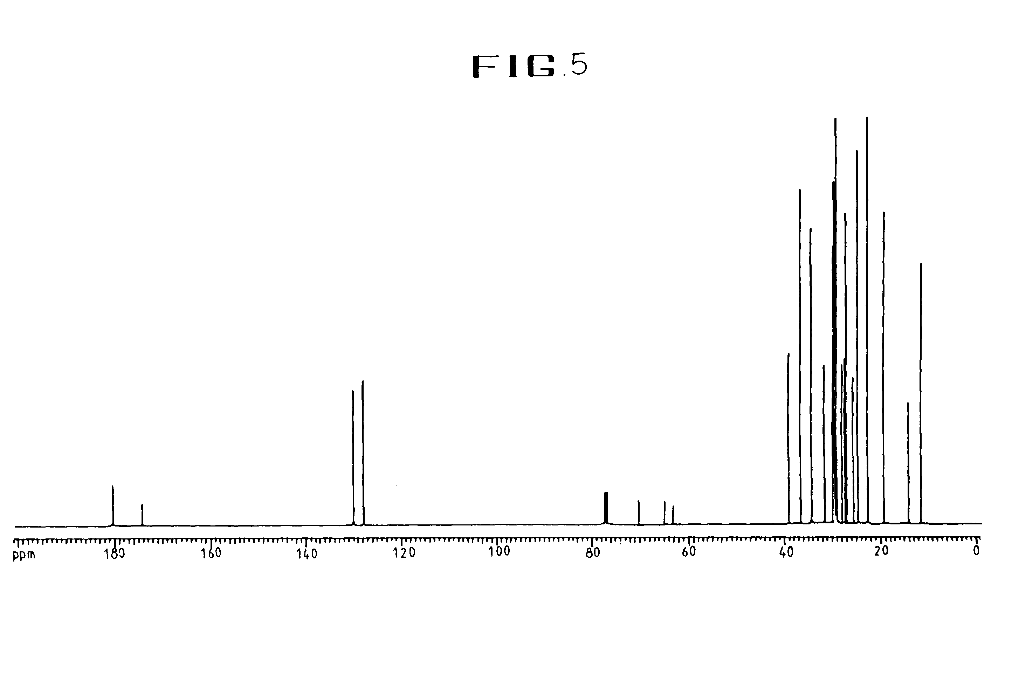

1. Thin-layer chromatography was conducted on Merck & Company No. 5642 HPTLC silica gel plate with the development of chloroform-methyl alcohol, 19:1, Antibiotic AL072 samples were spotted on the silia gel plate and developed in a sealed container. After development, an aqueous 10% sulfuric acid solution containing 5% ammonium molybdate and ceric ammonium sulfate was sprayed on the silica gel plate which was then heated to 100°C for 10 minutes to develop a colour. As a result of this, the Rf value of antibiotic AL072 was 0.58.
2. Antibiotic AL072 is soluble in alcohols such as methyl alcohol, ethyl alcohol, isopropyl alcohol, etc., and organic solvents such as ethyl acetate, chloroform, etc., whereas it is not or poorly soluble in water.
3. Antibiotic AL072 is very stable in aqueous solutions with pH values between pH 2 and pH 12. The activity of antibiotic AL072 is unchanged by heat of 95°C for 1 hour.
4. As a result of ultraviolet spectrometry, antibiotic AL072 in methyl alcohol is shown to have a maximum absorption in 242nm(Fig. 1).
5. The infrared absorption spectrum of antibiotic AL072 in potassium bromide using Bruker FT-IR spectrometer is shown in Fig. 2.
6. The proton unclear magnetic resonance spectrum of antibiotic AL072 in CDCl₃ using Bruker ARX400 spectrometer is shown in Fig. 4. H-NMR δ (400MHz): 0.86(3H, m); 0.87(3H, m); 0.88(3H, m); 0.91(3H, m); 1.29(43H, m); 1.61(4H, m), 2.03(2H, m); 2.31(4H, m); 2.75(1H, m); 3.62(1H, dd, 12Hz, 6Hz); 3.72(1H, dd, 4Hz, 12Hz); 3.96(1H, m); 4.14(2H, m); 5.35(4H, m) ppm
7. The carbon nuclear magnetic resonance spectrum of antibiotic AL072 in CDCl₂ using Bruker ARX400 spectrometer is shown in Fig. 5. C-NMR δ (100MHz); 12.01(q); 14.67(q); 19.85(q); 23.23(q); 23.28(q); 25.34(t); 25.36(t); 26.29(t); 27.78(t); 27.84(t); 27.87(t); 28.10(t); 28.63(d); 29.72(t); 29.75(t); 29.82(t); 29.93(t); 30.02(t); 30.17(t); 30.26(t); 30.29(t); 30.33(t); 30.36(t); 30.38(t); 30.62(t); 30.69(t); 32.20(t); 34.79(t); 34.81(t); 35.08(d); 37.33(t); 39.75(t); 63.90(t); 65.95(t); 70.96(d); 128.50(d); 128.70(d); 130.60(d); 130.70(d); 174.9(s); 181.00(s)ppm.

### Biological Activity of Antibiotic substance AL072

The effects of antibiotic substance AL072 obtained according to the above procedures on growth of various microbes were shown in Table V below. It is evident that while the antibiotic substance AL072 is powerfully active against *Legionella pheumophila,* it is not or only silightly active against the other microbes tested.

**Table V**

| Microorganism | Diameter of ring produced by inhibition of growth (mm) |
|---|---|
| *Legionella pneumophila* ATCC 33152 | 108 |
| *Staphylococcus aureus* | 11 |
| *Streptococcus pyogenes* | - |
| *Streptococcus* | 20 |
| *Streptococcus agalactiae* | - |
| *Streptococcus equi* | 21 |
| *Streptococcus durans* | 24 |
| *Listeria monocytogenes* | 12 |
| *Corynebacterium diphtheriae* | 9 |
| *Bacillus subtilis* | 8 |
| *Bacillus megaterium* | 6 |
| *Escherichia coli* | 6 |
| *Citrobacter freundii* | - |
| *Salmonella typhimurium* | 14 |
| *Shigella flexneri* | - |
| *Klebsiella pneumoniae* | - |
| *Klebsiella oxytoca* | - |
| *Klebsiella aerogenes* | - |
| *Enterobacter cloacae* | - |
| *Enterobacter aerogenes* | 9 |
| *Serratia marcescens* | - |
| *Proteus mirabilis* | - |
| *Proteus vulgaris* | 12 |
| *Proteus rettgeri* | - |
| *Proteus morgani* | - |
| *Pseudomonas aeruginosa* | - |
| *Pseudomonas cepacia* | - |

The susceptibility of *Legionella* sp. to antibiotic AL072 is tested by the well-known paper disc method. The preculture of *Legionella* sp. is prepared in accordance with a conventional manner by using a BCYE medium which consists of the following ingredients:

| a. Plate | |
|---|---|
| Yeast Extract | 11.5g |
| Activated Charcoal | 1.5g |
| ACES | 6.0g |
| α-Ketoglutaric Acid | 1.0g |
| Bacto Agar | 17.0g |
| Distilled Water (D.W.) | 1.0L |
| pH | 7.1-7.2(with 10M KOH) |
| | |

| b. Enrichment | |
|---|---|
| L-Cystein-HCl | 0.2g |
| Ferric Pyrophosphate | 0.125g/5ml of D.W. |
| | |

The BCYE medium is sterilized at 1 atmosphere, 121°C for 15 minutes before it is cooled to 50-55°C. The preculture of *Legionella* sp. is diluted with 0.9% saline (NaCl 9.0g + D.W. 1L) in a final concentration of 5 x 106 - 10⁷ colony forming unit/ml (cfu/ml). The dilution is dispensed and poured by 10ml into each petri-dish to form test plates. Paper discs with a diameter of 6mm are infiltrated with 20 µl of antibiotic AL072 and then well dried. The dried disc is placed on the prepared plate, which is then cultivated in 37°C incubator for 24 hours. The size of ring occurring by inhibition of growth is measured with the use of a vernier caliper.

The susceptibility test of *Streptococcus* sp. is conducted in accordance with similar procedures as above described, provided that the following medium are used:

| a. Mueller Hinton Medium(Difco) | |
|---|---|
| Beef infusion | 300.0g |
| Bacto Casamino Acids | 17.5g |
| Starch | 1.5g |
| Bacto Agar | 17.0g |

| b. Tryptic Soy Broth(Difco) | |
|---|---|
| Bacto Tryptone pancreatic digest of soybean meal | 17.0g |
| Bacto Soytone papic digest of soybean meal | 3.0g |
| Bacto Dextrose | 2.5g |
| Sodium Chloride | 5.0g |
| Dipotassium Phosphate 10% horse serum | 2.5g |
| | |

The susceptibility of the other aerobic or anaerobic microbes is tested by similar procedures to *Streptococcus* sp., except that the horse serum is unnecessary.

## Claims

1. A compound of the formula

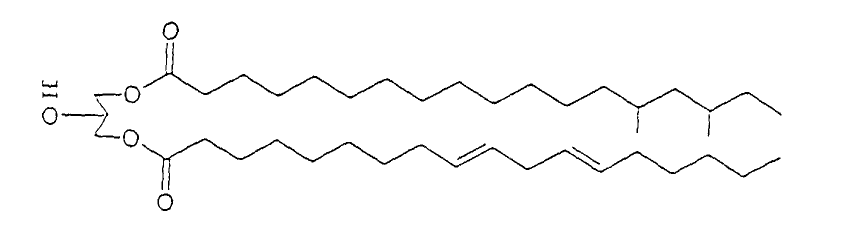

2. A microorganism *Streptomyces* sp. AL91 producing the *Legionella* specific antibiotic compound according to claim 1.

3. A process for producing the *Legionella* specific antibiotic compound according to claim 1 which process comprises cultivating the microorganism *Streptomyces* sp. AL91, extracting the cultural broth with isopropyl alcohol and ethyl acetate, and purifying the extracts by chromatography on column filled with octadecyl silica gel and in turn preparative high pressure liquid chromatography to obtain the said antibiotic compound.

4. A process according to claim 3, wherein said chromatography is performed by using said column filled with octadecyl silica gel using 70% EtOH as eluent and in turn by preparative high pressure liquid chromatography (206 nm) on silica gel using acetonitrile-distilled water (68:32) at the rate of 30 mL/min and then high pressure liquid chromatography under the same conditions as the preparative HPLC to obtain the said antibiotic compound.

## Patentansprüche

1. Verbindung der Formel

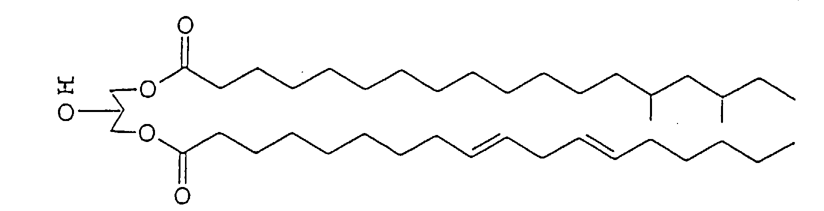

2. Mikroorganismus Streptomyces sp. AL91, der die Legionella-spezifische antibiotische Verbindung nach Anspruch 1 bildet.

3. Verfahren zur Herstellung der Legionella-spezifischen antibiotischen Verbindung nach Anspruch 1, das umfaßt
das Kultivieren des Mikroorganismus Streptomyces sp. AL91,
das Extrahieren der Kulturbrühe mit Isopropylalkohol und Ethylacetat und das Reinigen der Extrakte durch Chromatographie an einer Kolonne, die mit Octadecyl-Silicagel gefüllt ist, und anschließend durch präparative Hochdruck-Flüssigchromatographie zur Gewinnung der genannten antibiotischen Verbindung.

4. Verfahren nach Anspruch 3, bei dem die genannte Chromatographie durchgeführt wird unter Verwendung der genannten Kolonne, die mit Octadecyl-Silicagel gefüllt ist, unter Verwendung von 70 %igem EtOH als Eluierungsmittel und anschließend durch präparative Hochdruck-Flüssigchromatographie (206 nm) an Silicagel unter Verwendung von Acetonitril/destilliertem Wasser (68/32) bei einer Geschwindigkeit von 30 ml/min und dann durch Hochdruck-Flüssigchromatographie unter den gleichen Bedingungen wie die präparative HPLC zur Gewinnung der genannten antibiotischen Verbindung.

## Revendications

1. Composé de formule :

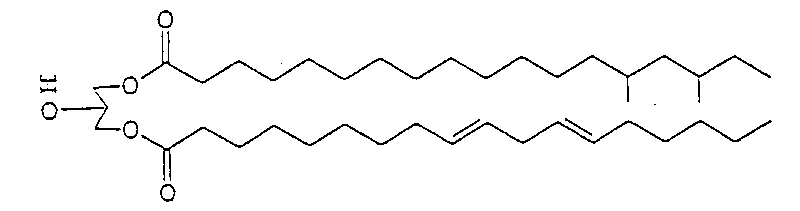

2. Microorganisme *Streptomyces* sp. AL91 produisant le composé antibiotique spécifique *Legionella* suivant la revendication 1.

3. Procédé pour la production du composé antibiotique spécifique *Legionella* suivant la revendication 1, lequel procédé comprend la culture du microorganisme *Streptomyces* sp. AL91, l'extraction du bouillon de culture avec de l'alcool isopropylique et l'acétate d'éthyle, et la purification des extraits par chromatographie sur colonne garnie de gel d'octadécyl silice et ensuite par chromatographie liquide haute pression préparative pour obtenir ledit composé antibiotique.

4. Procédé suivant la revendication 3, dans lequel ladite chromatographie est réalisée par utilisation de ladite colonne garnie de gel d'octadécyl silice avec de l'EtOH à 70% utilisé comme éluant, puis par chromatographie liquide haute pression préparative (206 nm) sur gel de silice avec un mélange 68:32 d'acétonitrile et d'eau distillée utilisé comme éluant au débit de 30 ml/mn et ensuite par chromatographie liquide haute pression dans les mêmes conditions que celles de la HPLC préparative pour obtenir ledit composé antibiotique.
